# EUROPEAN PATENT SPECIFICATION

(11) **EP 3 491 929 B1**
(45) Date of publication and mention of the grant of the patent: **21.07.2021**
(21) Application number: 18209212.2
(22) Date of filing: 29.11.2018
(51) Int. Cl.: A23F 5/24, A61K 31/35, A61K 31/522, A61K 36/00, A23L 33/105

(54) **AMELIORATIVE EFFECTS OF A WHOLE COFFEE FRUIT EXTRACT ON AGE-RELATED NEURODEGENERATIVE DISEASE**
VERBESSERNDE WIRKUNG DES EXTRAKTS DER GANZEN KAFFEEFRUCHT AUF ALTERSBEDINGTE NEURODEGENERATIVE ERKRANKUNGEN
EFFETS D'AMÉLIORATION D'UN EXTRAIT DE FRUITS DE CAFÉ ENTIERS SUR UNE MALADIE NEURODÉGÉNÉRATIVE

(30) Priority: 29.11.2017 US 201762591998 P
(43) Date of publication of application: 05.06.2019
(73) Proprietor: Lytone Enterprise, Inc., New Taipei City 221-80, R.O.C. (TW)
(72) Inventor: CHANG, William Tienhung, R.O.C. 22180 NEW TAIPEI CITY (TW); TSENG, WEITING, R.O.C. 22180 NEW TAIPEI CITY (TW); CHEN, MINGHUI, R.O.C. 22180 NEW TAIPEI CITY (TW); CHAN, Yin-Ching, TAICHUNG CITY 43301 (TW)
(74) Representative: De Clercq & Partners

(56) References cited:
- WO-A1-2013/049523
- WO-A1-2017/040810
- US-A1- 2016 038 557
- US-A1- 2017 007 658
- US-A1- 2017 327 776
- HEIMBACH J T ET AL: "Safety studies on products from whole coffee fruit", FOOD AND CHEMICAL TOXICOLOGY, PERGAMON, GB, vol. 48, no. 8-9, 1 August 2010 (2010-08-01), pages 2517-2525, XP027166469, ISSN: 0278-6915 [retrieved on 2010-06-18]
- TANIA REYES-IZQUIERDO ET AL: "Modulatory effect of coffee fruit extract on plasma levels of brain-derived neurotrophic factor in healthy subjects", BRITISH JOURNAL OF NUTRITION, vol. 110, no. 03, 14 January 2013 (2013-01-14), pages 420-425, XP055282155, UK ISSN: 0007-1145, DOI: 10.1017/S0007114512005338
- TANIA REYES-IZQUIERDO ET AL: "Stimulatory Effect of Whole Coffee Fruit Concentrate Powder on Plasma Levels of Total and Exosomal Brain-Derived Neurotrophic Factor in Healthy Subjects: An Acute Within-Subject Clinical Study", FOOD AND NUTRITION SCIENCES, vol. 04, no. 09, 1 January 2013 (2013-01-01), pages 984-990, XP055553893, ISSN: 2157-944X, DOI: 10.4236/fns.2013.49127
- NARGES TAJIK ET AL: "The potential effects of chlorogenic acid, the main phenolic components in coffee, on health: a comprehensive review of the literature", EUROPEAN JOURNAL OF NUTRITION, vol. 56, no. 7, 1 October 2017 (2017-10-01), pages 2215-2244, XP055553177, DE ISSN: 1436-6207, DOI: 10.1007/s00394-017-1379-1
- CAO CHUANHAI ET AL: "Caffeine synergizes with another coffee component to increase plasma GCSF: linkage to cognitive benefits in Alzheimer's mice", JOURNAL OF ALZHEIMER'S DIS, IOS PRESS, NL, vol. 25, no. 2, 1 January 2011 (2011-01-01), pages 323-335, XP009165440, ISSN: 1387-2877

## Description

### Field of the Invention

The invention provides a method of extracting whole coffee fruit, an extract obtained from the method, and method of ameliorating age-related neurodegenerative diseases using said extract.

### Background of the Invention

Coffee fruits are mainly produced in Africa and the Middle East. Reports indicate that in Africa and Asia, the peel and pulp of coffee fruits are used for fermentation into wines or directly as food for chewing. In Yemen, the peel of coffee fruits is mixed with spices and boiled for making a drink which the local people call "qishr" (Beckman, I. 2000, Journal of the Weston A. Price Foundation). On the other hand, the results of a toxicology test in rats fed with whole coffee fruit extract for 90 days demonstrate that the highest non-observed effect level (NOEL) is 3446 mg/kg bw/day (male rat) and 4087 mg/kg bw/day (female rat) (Heimbach, J.T, et al., Food Chem. Toxicol. 48, 2517-2525). The above references prove that whole coffee fruit is safe for eating and thus can be prepared as food.

Coffee fruits contain high levels of phenol antioxidants. However, in traditional coffee processing, in order to avoid contamination by mycotoxins produced due to rapid decomposition after harvest, the peel and pulp are removed immediately or discarded or used as fertilizer, and extracts are often prepared from the coffee beans only, such as for example disclosed in US2017/0007658. In fact, the peel and pulp are enriched with nutrients and antioxidants (Napolitana A., et al., J. Agric. Food Chem. 55. 10499-10504). References prove that the water extract of coffee fruits contains more than 85% of polyphenols and that every 100g extract comprises 0.6 to 1.5 million units of oxygen radical absorption capacity (ORAC) (Nagasawa, H., et al., Anticancer Res. 15. 141-146). Coffee fruits were also proved as having the potential in immune-modulation and inhibiting tumor growth (Kobayashi, T., et al., Anticancer Res. 18. 187-190; Nagasawa, H., et al., Anticancer Res. 15. 141-146). Also extracts from de-beaned coffee fruits were shown to contain highly levels of antioxidants and nutrients (WO2017/040810).

Aging usually accompanies degeneration in learning memories, which may be caused due to factors such as oxidative stress, neuron cell damage, and neurotrophy factor degeneration. Neurotrophy factors are a family composed of structurally related proteins and can modulate the survival, differentiation, development, and function of neurons of the peripheral and central nervous systems. They are also important for the synapse to modulate neuron signal transduction and function (Huang et al., 2001). Brain-derived neurotrophic factors (BDNF) are the most abundant neurotrophic factors in the brain. Researchers have proven that micro-glial cells secrete large amounts of BDNF and glial cell line-derived neurotrophic factors (GDNF) in damaged brain regions as a mechanism of neuron protection (Imaiet al., 2007; Suzukiet al., 2001). BDNF modulates neurotransmitters, and participates in the processes of neuron growth, differentiation and remodeling, for example, memory and learning controlled by the hippocampus. Protein concentration of BDNF is modulated by neuron activities. When neuron activities increase, the signal transduction pathway of cAMP-response element binding protein (cAMP-CREB) is activated accordingly, wherein CREB is an important transcription factor that enhances the expression level of BDNF (Lonze et al., 2002). The α subunit of eukaryotic initiation factor 2 (eIF2α) is a key subunit during the translation initiation process in eukaryotic organisms, which inhibits the initiation of translation when being phosphorylated, reduces overall protein synthesis, and affects the remodeling of synapses and stabilization of neurons. p-eIF2α also increases activating transcription factor 4 (ATF4) and functions as an inhibitor of CREB, so that subsequent transcription is inhibited. In addition, through gene-specific translation, it can increase the synthesis of BACE-1, drive amyloid precursor protein (APP) to the pathway of forming amyloids, and thus collaboratively causes neuron degeneration.

Over-inflammation will induce deposition of Amyloid-β (Aβ), and Aβ simultaneously activates inflammation reactions, which thus forms a vicious circle. NLRP3 is a member of the NLR family and a complex that detects invasion of foreign micro-organisms and activates inflammation reactions. It converts the cytokine pro-interleukin 1 beta (pro-IL-1β) into an active IL-1β through activating caspase-1 for defending against foreign substances. On the other hand, too much IL-1β in the neuron cells will induce the production of COX-2 and aggravate overall inflammation reactions. Eventually, neuronal inflammation may be caused, leading the neuron to apoptosis.

Alzheimer's disease (AD) is the most common age-related neuron degenerative disease which is characterized by the deposition of Aβ plaques in the brain. Aggregation of Aβ produces large amounts of peroxides, leading to oxidative stress and inflammation and promoting degeneration of neurotrophic factors. Eventually, it may cause death of neuron cells through autophagy or the apoptosis pathway, and causes deficiency in learning and memory abilities (Holtzman et al., 2011).

Over-accumulation of Aβ deposition could induce oxidative stress, inflammatory response, and degeneration of BDNF thereby causing age-related neurodegenerative diseases such as AD. Despite the versatile function of whole coffee fruit extract or caffeine, such as a modulatory effect of whole coffee fruit extract on plasma levels of BDNF (Reyes-Izquierdo et al., 2013) or a general positive effect of caffeine on brain health (WO2013/049523), the effects of whole coffee fruit extract on Aβ deposition and AD remain unknown.

In the present invention, the effects of a whole coffee fruit extract on neurodegenerative disease and potential protective efficacy against Alzheimer's disease were confirmed. These results indicate that whole coffee fruit extract may have potential therapeutic applications on various neurodegenerative disorders and offer a reference for developing anti-aging health foods in the future.

### Summary of the Invention

It is found in the present invention that a whole coffee fruit extract is capable of decreasing the expression and deposition of Aβ in the brain, and modulating factors relating to AD. Therefore, the present disclosure provides a novel method for producing a whole coffee fruit extract, a whole coffee fruit extract produced by said method, and said whole coffee fruit extract for use in a method of ameliorating age-related neurodegenerative diseases, such as Alzheimer's disease, Parkinson's disease, ischemic dementia, and Huntington's disease.

As set out in the appended claims, the whole coffee fruit extract is extracted by 25 to 30% ethanol.

As set out in the appended claims, the whole coffee fruit extract is extracted under the temperature of 70 to 80°C.

As set out in the appended claims, the whole coffee fruit extract is extracted for 3.5 to 4 hours.

As set out in the appended claims, the whole coffee fruit extract is extracted under a solid to liquid ratio of 1:10 to 1:5.

As set out in the appended claims, the present invention discloses a method for producing a whole coffee fruit extract, comprising the steps of: (a) providing freshly collected whole coffee fruit, drying the whole coffee fruit so that the water content is 20% or less; (b) extracting the dried whole coffee fruit of step (a) with 25 to 30% ethanol at a solid to fluid ratio of 1:10 to 1:5 at 70 to 80°C for 3.5 to 4 hours; and (c) recovering the extract of step (b), wherein the whole coffee fruit includes seed of the fruit.

The present disclosure also relates to a whole coffee fruit extract produced by the method described above. In a preferred embodiment, the whole coffee fruit extract comprises 5-O-caffeoylquinic acid (5-CQA), chlorogenic acid (CA) and procyanidine. In an embodiment, the whole coffee fruit extract comprises CA and procyanidine.

The present invention further relates to a whole coffee fruit extract as described above for use in a method as set out in the appended claims comprising administering to a subject in need thereof a therapeutically effective amount of a whole coffee fruit extract described above. In an embodiment, the therapeutic effective amount of the whole coffee fruit extract is about 50 to about 400 mg/kg, preferably about 100 to about 300 mg/kg, more preferably about 200 mg/kg. In an embodiment, Aβ, *p*-eIF2α, BACE-1, *p*-CREB, BDNF, NLRP3, caspase-1, IL-1β, and COX-2 in the subject are regulated. In an embodiment, *p*-eIF2α, BACE-1, Aβ, NLRP3, caspase-1, IL-1β and COX-2 are down-regulated and *p*-CREB and BDNF are up-regulated. In an embodiment, Aβ deposition in Alzheimer's disease is reduced. In an embodiment, the age-related neurodegenerative diseases are selected from Alzheimer's disease, Parkinson's disease, ischemic dementia, and Huntington's disease. In an embodiment, the age-related neurodegenerative disease is Alzheimer's disease.

The present invention is described in detail in the following sections. Other characterizations, purposes and advantages of the present invention can be easily found in the detailed descriptions and claims of the invention.

### Brief Description of the Drawings

Figures 1A and 1B demonstrate the ratio of expression level of *p*-eIF2α to β-actin in the hippocampus and cortex, respectively, of 3-month old male SAMP8 mice after feeding with different chews for 12 weeks (a: Control, b: 200 mg/kg whole coffee fruit extract).
Figures 2A and 2B demonstrate the ratio of expression level of BACE-1 to β-actin in the hippocampus and cortex, respectively, of 3-month old male SAMP8 mice after feeding with different chews for 12 weeks (a: Control, b: 200 mg/kg whole coffee fruit extract).
Figures 3A and 3B demonstrate the percentage of area deposition of Aβ in the hippocampus and whole brain, respectively, of 3-month old male SAMP8 mice after feeding with different chews for 12 weeks (a: Control, b: 200 mg/kg whole coffee fruit extract).
Figures 4A and 4B demonstrate the ratio of expression level of *p*-CREB to CREB in the hippocampus and cortex, respectively, of 3-month old male SAMP8 mice after feeding with different chews for 12 weeks (a: Control, b: 200 mg/kg whole coffee fruit extract).
Figures 5A and 5B demonstrate the ratio of expression level of BDNF to β-actin in the hippocampus and cortex, respectively, of 3-month old male SAMP8 mice after feeding with different chews for 12 weeks (a: Control, b: 200 mg/kg whole coffee fruit extract).
Figures 6A and 6B demonstrate the ratio of expression level of NLRP3 to β-actin in the hippocampus and cortex, respectively, of 3-month old male SAMP8 mice after feeding with different chews for 12 weeks (a: Control, b: 200 mg/kg whole coffee fruit extract).
Figures 7A and 7B demonstrate the ratio of expression level of Caspase-1 p20 to β-actin in the hippocampus and cortex, respectively, of 3-month old male SAMP8 mice after feeding with different chews for 12 weeks (a: Control, b: 200 mg/kg whole coffee fruit extract).
Figures 8A and 8B demonstrate the ratio of expression level of IL-1β to β-actin in the hippocampus and cortex, respectively, of 3-month old male SAMP8 mice after feeding with different chews for 12 weeks (a: Control, b: 200 mg/kg whole coffee fruit extract).
Figures 9A and 9B demonstrate the ratio of expression level of COX-2 to β-actin in the hippocampus and cortex, respectively, of 3-month old male SAMP8 mice after feeding with different chews for 12 weeks (a: Control, b: 200 mg/kg whole coffee fruit extract).
Figure 10 shows the results of immunohistochemical (IHC) staining for the levels of Aβ deposition in the brain of 3-month old male SAMP8 mice after feeding with normal chew (A) and the whole coffee fruit extract (200 mg/kg) of the present invention (B) for 12 weeks.

### Detailed Description of the Invention

Unless otherwise defined herein, scientific and technical terms used in connection with the present invention shall have the meanings that are commonly understood by those of ordinary skill in the art. The meaning and scope of the terms should be clear; however, in the event of any latent ambiguity, definitions provided herein take precedence over any dictionary or extrinsic definition.

As utilized in accordance with the present disclosure, the following terms, unless otherwise indicated, shall be understood to have the following meanings.

The term "whole coffee fruit" as used herein refers to the entire fruit of the coffee tree (Coffea spec.) in which the exocarp and the outer mesocarp (i.e., the pulp) surround the inner mesocarp (i.e. the mucilage) and endocarp (i.e., the hull), which in turn surround the seeds (i.e., the beans). Thus, the term whole coffee fruit specifically refers to a whole coffee fruit, which includes the seed of the fruit.

The whole coffee fruit extract is prepared by the method disclosed herein. In a preferred embodiment, the whole coffee fruit extract which is prepared by the method disclosed herein is that under the product name, Anthochlorogin.

The term "ameliorating" as used herein refers to delaying the onset of the symptoms of a susceptible subject or reducing the occurrence of a disease, reducing and/or improving the symptoms of a susceptible subject or increasing the survival rate of the subject with certain lethal disorders or conditions..

The term "age-related neurodegenerative diseases" as used herein refers to the neurodegenerative diseases that progress as age increases, for example, brain function and cognition decline with aging. For example, such diseases include Alzheimer's disease, Parkinson's disease, ischemic dementia, and Huntington's disease.

The term "subject" as used herein denotes animals, especially mammals. In one preferred embodiment, the term "subject" denotes humans.

The term "therapeutically effective amount" as used herein refers to the amount of an active ingredient used alone or in combination with other treatments/medicaments for treating age-related neurodegenerative diseases that shows therapeutic efficacy.

Unless otherwise required by context, singular terms shall include the plural and plural terms shall include the singular.

The inventors of the invention found that a whole coffee fruit extract can enhance the expression of *p*-CREB and BDNF and decrease the expression of *p*-eIF2α, BACE-1, Aβ, NLRP3, caspase-1, IL-1β and COX-2. As these parameters may be associated with age-related neurodegenerative disease, the present invention may thus provide a novel therapy for age-related neurodegenerative disease. In a preferred embodiment, the age-related neurodegenerative disease is Alzheimer's disease.

In addition to the data on the enzymes, *in situ* data showing the reduced deposition of Aβ in the brain of the mice further evidence the efficacy of the whole coffee fruit extract in diseases associated with Aβ deposition. In an embodiment, the disease associated with Aβ deposition is Alzheimer's disease.

Having now generally described the invention, the same may be more readily understood through reference to the following examples, which provide exemplary protocols for the production of the whole coffee fruit extract of the invention and its use in the amelioration of age-related neurodegenerative disease. The examples are offered for illustrative purposes only, and are not intended to limit the scope of the present invention in any way. Efforts have been made to ensure accuracy with respect to numbers used (e.g., amounts, temperatures, etc.), but some experimental error and deviation should, of course, be allowed for.

### Examples

### Example 1: Preparation of whole coffee fruit extract

Coffee (*Coffee Arabica*) berry raw materials were obtained from the coffee garden of farmers or agricultural production and marketing groups in Tainan, Pingtung, Hualien and Taitung. The coffee fruits (fresh fruit) were sterilized after receipt so as to reduce surface microbes. Then, the coffee fruits were delivered to the lab by a preservation technology. Right after arrival, the coffee fruits were subjected to cold wind or freeze drying so that the fresh fruits were dried to reach a water content of 20% or less. After that, the coffee fruits were stored or grounded into powder for extraction.

During extraction, the whole coffee fruit powders were dissolved in ethanol for extraction. The extract was subjected to high performance liquid chromatography (HPLC) for analysis of possible active ingredients. The results reveal that the extract comprises 5-CQA, CA and procyanidine. The extract was freeze-dried and a brown to deep brown powder was obtained, which is named as Anthochlorogin. Before evaluation on the biological activities of the extract, the dried product was dissolved in corn oil and adjusted to desired concentrations in the chew.

### Example 2: Experimental design and composition of feedstuff

Senescence accelerated mice P8 (SAMP8) was used as the animal model for evaluating amelioration of Alzheimer's disease. This mice model is characterized in having age-related memory defects. Whether whole coffee fruit extract has efficacy in improving learning and memory capacities and the effect on related molecular mechanism were explored. 3-month old SAMP8 were raised in 30(W)×20(D)×10(H) cm transparent plastic cages. The temperature was kept at 22±2°C, relative humidity kept at 65±5% and the room had automatically controlled light periods, where 7:00-19:00 was the dark period and 19:00-7:00 was the light period. Before testing, animals were accommodated for 3-5 days. At the beginning of the experiment, mice were separated into a control group taking standard diet (20% Casein, 5 % corn oil, 1% vitamin mixture (AIN93-G), 5% mineral mixture (AIN93-G), 2% cellulose powder, and 2.5% choline) and an experimental group taking an additional 200 mg/kg whole coffee fruit extract. Said 200 mg/kg is a human daily dose and was converted to mice dose for administration. Feedstuff and water were taken freely. During the experiment, the amounts of food intake and body weight were recorded. After feeding for 12 weeks, the mice were sacrificed and brain samples were subjected to analysis for amyloid β (Aβ), phospho-eukaryotic initiation factor 2α (p-eIF2α), beta-site amyloid precursor protein-cleaving enzyme-1 (BACE-1), brain-derived neurotrophic factor (BDNF) and phospho-cAMP response element-binding protein (p-CREB). Levels of the inflammation markers NLRP3, caspase-1, IL-1β, and COX2 were also measured.

### Example 3: Western blot analysis

Brain tissue samples were homogenized. According to the molecular weight of the target protein to be observed, different concentrations of SDS-PAGE gels were prepared for electrophoresis. The samples were heated at 100°C and sequentially loaded into the wells of the SDS-PAGE gels. Separation was conducted at 65V 100 mins and 100V 80 mins for the target protein to be able to run to the desired position. After electrophoresis, SDS-PAGE gels and PVDF membranes were placed in a cassette for transferring the target protein. The transfer was conducted in a 4°C cold room.

After completion of transfer, the PVDF membrane was cut according to the molecular weight of the target protein and placed in small box for blocking for 1 hour. The membrane was then washed by wash buffer on a shaker for several times. After that, primary antibody solution was added to soak the membrane on a shaker in a 4°C cold room and recovered after 8-16 hours of incubation. Secondary antibody solution was then added to soak the membrane on a shaker at room temperature for 1 hour. The PVDF membrane was then incubated with an enhanced chemiluminescence (ECL) substrate and placed in a chemiluminescence imaging system for taking photos of the images.

The data of each experimental group in this example were analyzed by SPSS software. The results are represented by mean ± S.E.M.. Differences of protein expression among each group were analyzed using one-way analysis of variance (one-way ANOVA). *P*<0.05 represents significant difference.

### Example 3.1: Analysis of the expression level of the Aβ related factor p-eIF2α

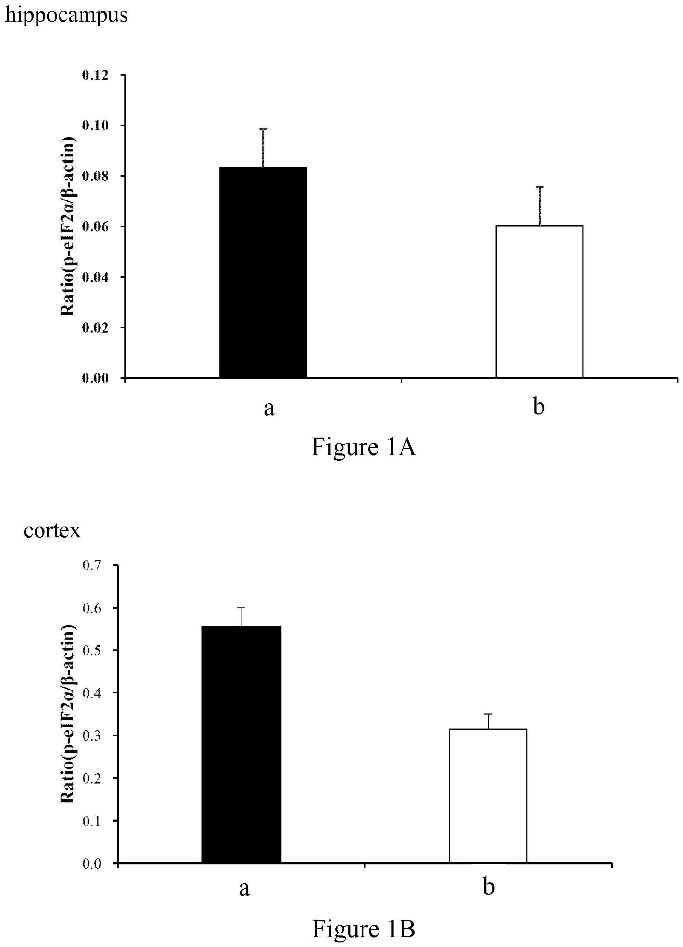

As shown in Figures 1A and 1B, in 3-month old male SAMP8 mice fed with different chews (a: Control, b: 200 mg/kg whole coffee fruit extract) for 12 weeks, the ratio of expression levels of *p*-eIF2α to β-actin in the hippocampus (Figure 1A) and cortex (Figure 1B) of the mice brain are decreased. The decreases reach statistical significance.

### Example 3.2: Analysis of the expression level of Aβ regulatory factor BACE-1

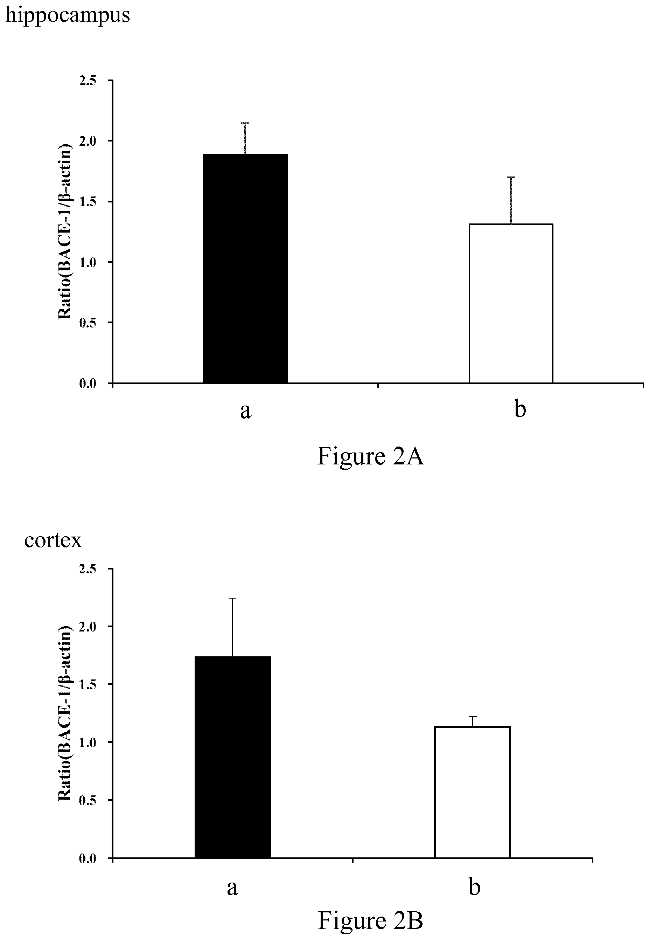

As shown in Figures 2A and 2B, in 3-month old male SAMP8 mice fed with different chews (a: Control, b: 200 mg/kg whole coffee fruit extract) for 12 weeks, the ratio of expression levels of BACE-1 to β-actin in the hippocampus (Figure 2A) and cortex (Figure 2B) of the mice brain are decreased. The decreases reach statistical significance.

### Example 3.3: Analysis of the deposition of Aβ in the brain

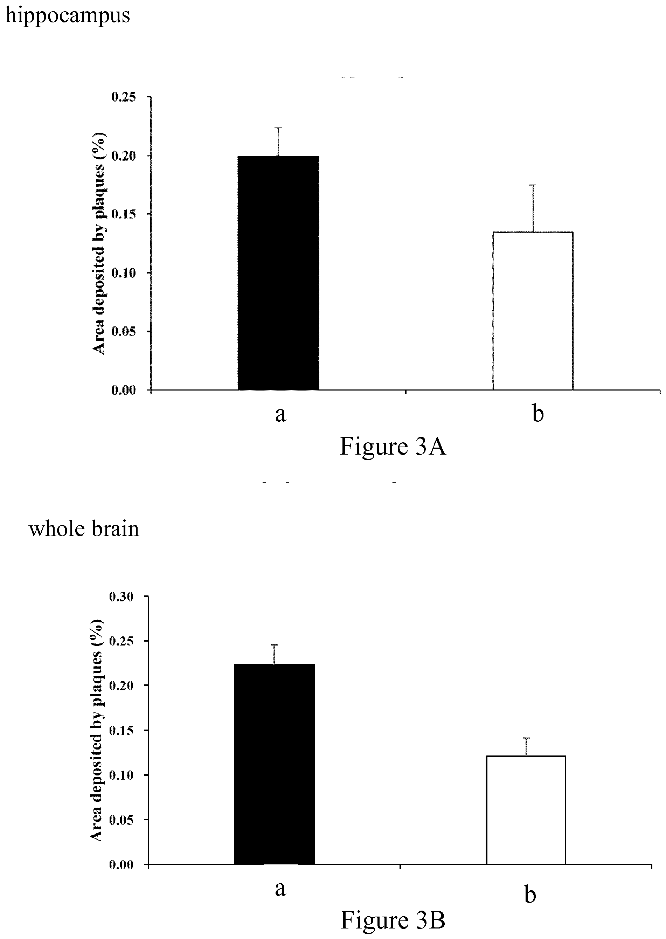

As shown in Figures 3A and 3B, in 3-month old male SAMP8 mice fed with different chews (a: Control, b: 200 mg/kg whole coffee fruit extract) for 12 weeks, the percentage of area deposition of Aβ in the hippocampus (Figure 3A) and whole brain (Figure 3B) of the mice brain are decreased. The decreases reach statistical significance.

### Example 3.4: Analysis of the expression level of neurotrophy factor p-CREB

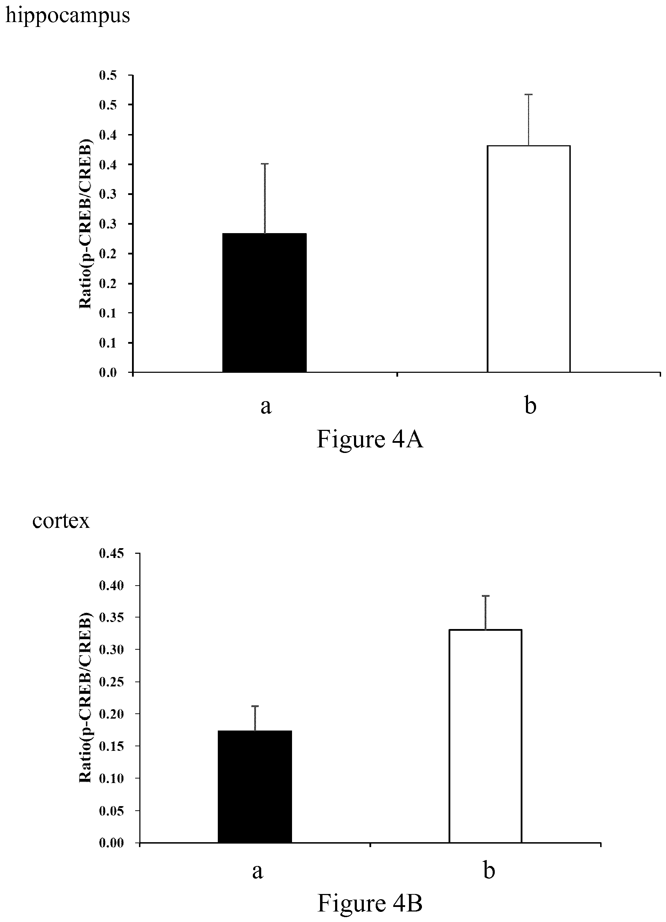

As shown in Figures 4A and 4B, in 3-month old male SAMP8 mice fed with different chews (a: Control, b: 200 mg/kg whole coffee fruit extract) for 12 weeks, the ratio of expression levels of *p*-CREB to CREB in the hippocampus (Figure 4A) and cortex (Figure 4B) of the mice brain are increased. The increases reach statistical significance.

### Example 3.5: Analysis of the expression level of neurotrophy factor BDNF

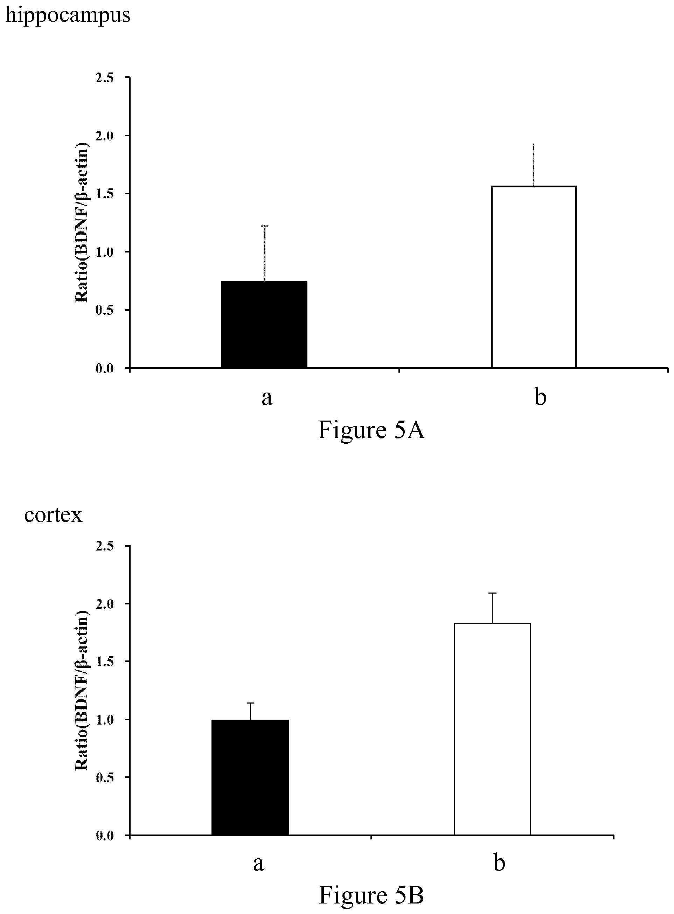

As shown in Figures 5A and 5B, in 3-month old male SAMP8 mice fed with different chews (a: Control, b: 200 mg/kg whole coffee fruit extract) for 12 weeks, the ratio of expression levels of BDNF to β-actin in the hippocampus (Figure 5A) and cortex (Figure 5B) of the mice brain are increased. The increases reach statistical significance.

### Example 3.6: Analysis of the expression level of inflammation related factor NLRP3

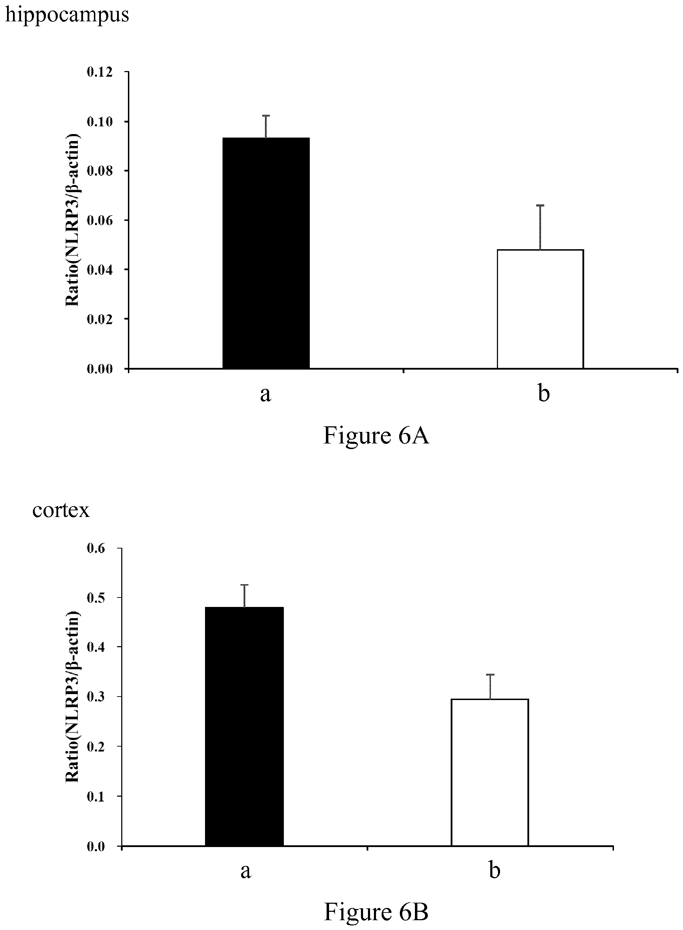

As shown in Figures 6A and 6B, in 3-month old male SAMP8 mice fed with different chews (a: Control, b: 200 mg/kg whole coffee fruit extract) for 12 weeks, the ratio of expression levels of NLRP3 to β-actin in the hippocampus (Figure 6A) and cortex (Figure 6B) of the mice brain are decreased. The decreases reach statistical significance.

### Example 3.7: Analysis of the expression level of inflammation related factor caspase-1

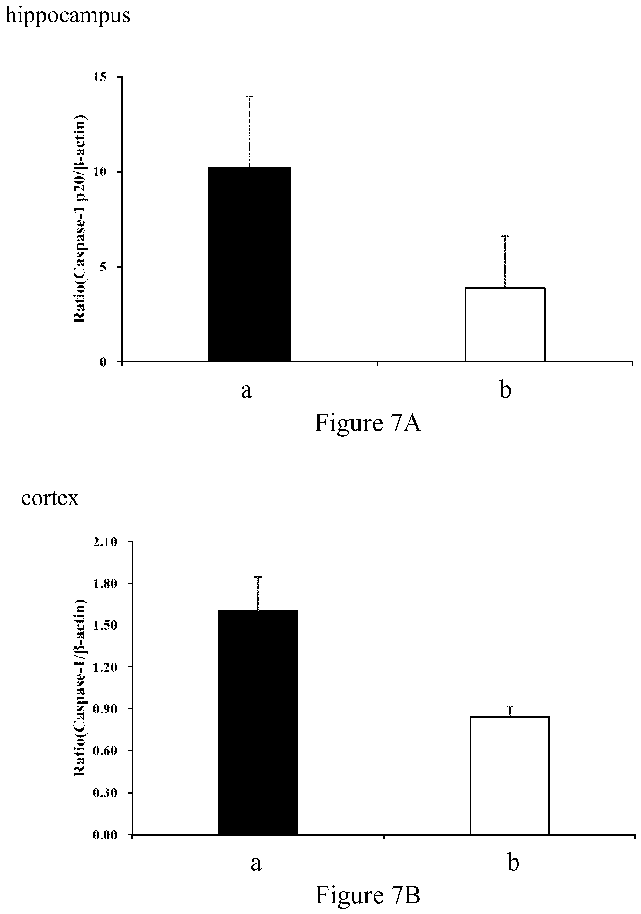

As shown in Figures 7A and 7B, in 3-month old male SAMP8 mice fed with different chews (a: Control, b: 200 mg/kg whole coffee fruit extract) for 12 weeks, the ratio of expression levels of caspase-1 p 20 to β-actin in the hippocampus (Figure 7A) and cortex (Figure 7B) of the mice brain are decreased. The decreases reach statistical significance.

### Example 3.8: Analysis of the expression level of inflammation related factor IL-1β

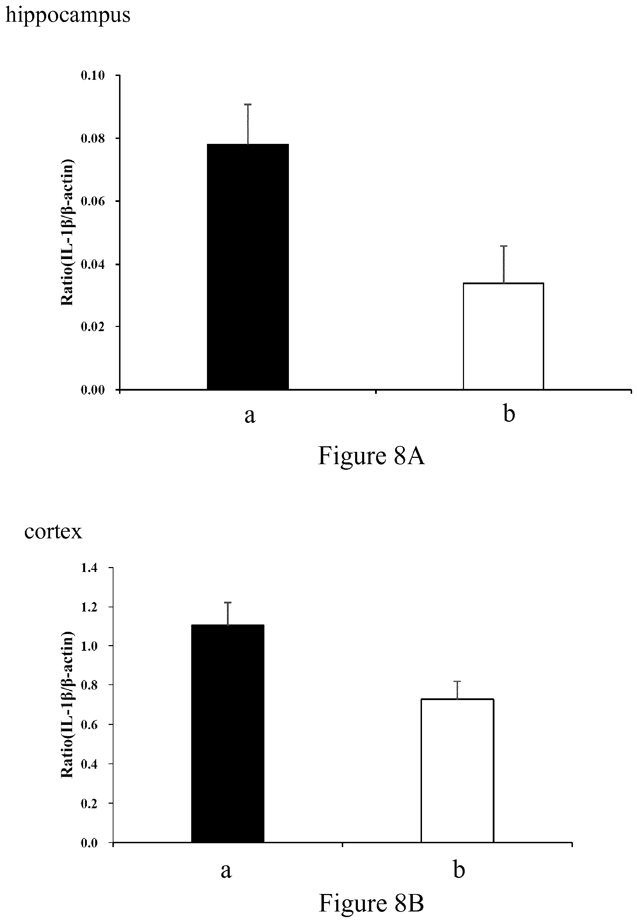

As shown in Figures 8A and 8B, in 3-month old male SAMP8 mice fed with different chews (a: Control, b: 200 mg/kg whole coffee fruit extract) for 12 weeks, the ratio of expression levels of IL-1β to β-actin in the hippocampus (Figure 8A) and cortex (Figure 8B) of the mice brain are decreased. The decreases reach statistical significance.

### Example 3.9: Analysis of the expression level of inflammation related factor COX-2

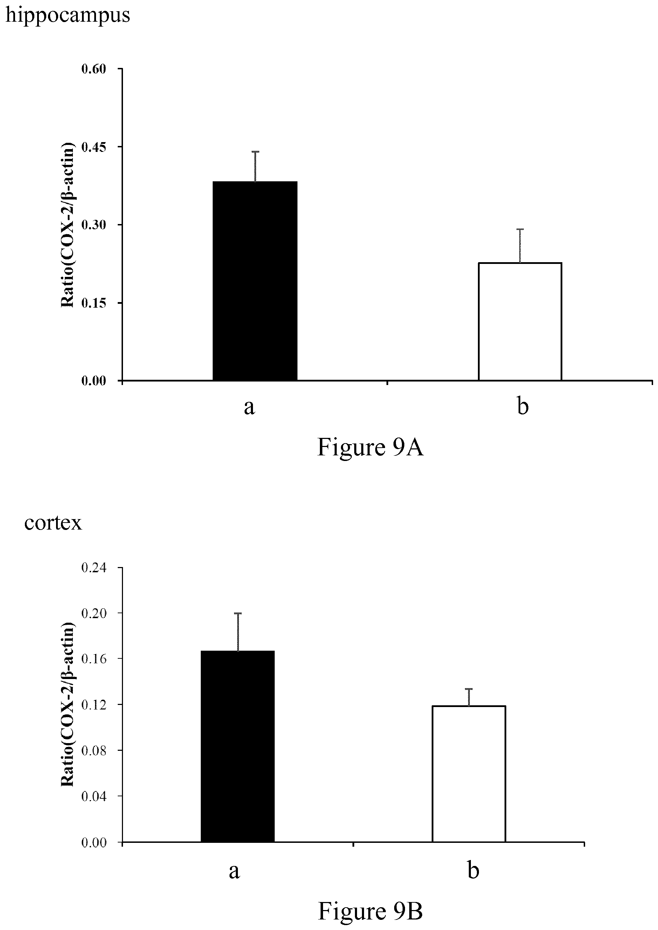

As shown in Figures 9A and 9B, in 3-month old male SAMP8 mice fed with different chews (a: Control, b: 200 mg/kg whole coffee fruit extract) for 12 weeks, the ratio of expression levels of COX-2 to β-actin in the hippocampus (Figure 9A) and cortex (Figure 9B) of the mice brain are decreased. The decreases reach statistical significance.

### Example 4: Immunohistochemical (IHC) staining for Aβ

After mice were sacrificed, brain tissues were obtained and immediately soaked in 10% formalin for paraffin embedding and tissue slicing (3-5 µm/slice). Before performing IHC staining, the slices were placed in xylene for dewaxing and treated with ethanol for removing xylene. The slices were soaked in Trilogy and heated at 121°C for 15 minutes to restore antigenicity followed by IHC staining. At first, goat serum-PBS solution was added for blocking. After that, diluted monoclonal anti-Aβ antibody was added and incubated at 4°C overnight. The next day, superenahncer solution was added for reaction at room temperature in a dark place followed by reaction with poly-HRP reagent at room temperature in the dark place. DAB was used for 1.5 minutes for colorification and then hematoxylin was used for counter stain. After completion, the slices were placed on an optical microscope to observe the density of a brownish substance which is equivalent to the amount of Aβ deposition.

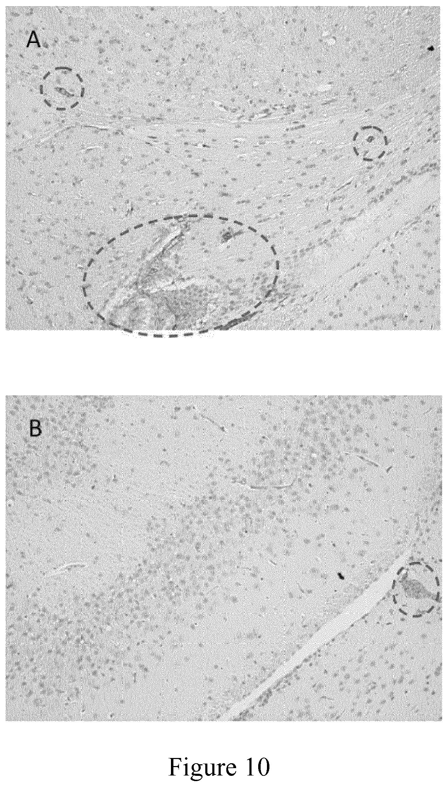

The results are shown in Figure 10 which represents the level of deposition of Aβ in the brain of 3-month old male SAMP8 mice fed with different diets for 12 weeks. Figure 10A is the result of the control group in which mice were fed with normal chew. Figure 10B is the result of the experimental group in which mice were fed with chew additionally added with 200 mg/kg whole coffee fruit extract of the present invention. The results in Figure 10 clearly demonstrate that the level of Aβ deposition in Figure 10A is obviously more intense than that in Figure 10B. This result indicates that mice fed with 200 mg/kg whole coffee fruit extract of the present invention have significantly reduced amount of Aβ deposition in the brain of SAMP8 mice. Thus, diseases or symptoms associated with Aβ deposition may be ameliorated in SAMP8 mice fed with the whole coffee fruit extract of the present invention.

Age-related neurodegenerative diseases may be caused due to oxidative stress, neuron cell damage, or decreased levels of neurotrophy factors. From the examples demonstrated above, it was surprisingly found that by ingesting the whole coffee fruit extract of the present invention, the levels of neurotrophy factors *p*-CREB and BDNF were increased, and *p*-eIF2α and BACE-1 that regulate Aβ were decreased. Eventually, decreased deposition of Aβ was observed. Further, the expression levels of inflammation factors NLRP3, caspase-1, IL-1β and COX-2 were decreased. Thus, the whole coffee fruit extract of the present invention, which may comprise chlorogenic acid (CA) and procyanidine, may be useful for ameliorating age-related neurodegenerative diseases.

Numerous modifications and variations of the invention as set forth in the above illustrative examples are expected to occur to those skilled in the art. Consequently, only such limitations as appear in the appended claims should be placed on the invention.

## Claims

1. A method of producing a whole coffee fruit extract, comprising the steps of:
(a) providing freshly collected whole coffee fruit, drying the whole coffee fruit so that the water content is 20% or less;
(b) extracting the dried whole coffee fruit of step (a) with 25 to 30% ethanol at a solid to liquid ratio of 1:10 to 1:5 at 70 to 80°C for 3.5 to 4 hours; and
(c) recovering the extract of step (b),
wherein the whole coffee fruit includes seed of the fruit.

2. A whole coffee fruit extract obtainable from the method comprising the steps of:
(a) providing freshly collected whole coffee fruit, drying the whole coffee fruit so that the water content is 20% or less;
(b) extracting the dried whole coffee fruit of step (a) with 25 to 30% ethanol at a solid to liquid ratio of 1:10 to 1:5 at 70 to 80°C for 3.5 to 4 hours; and
(c) recovering the extract of step (b),
wherein the whole coffee fruit includes seed of the fruit,
for use in a method for reducing Aβ deposition in the hippocampus of a subject in need thereof comprising administering to the subject a therapeutically effective amount of the whole coffee fruit extract.

3. The whole coffee fruit extract for use of claim 2, wherein the method increases expression levels of *p*-CREB and BDNF in the hippocampus and cortex of the subject and the method decreases the expression levels of *p*-eIF2α, BACE-1, Aβ, NLRP3, caspase-1, IL-1β and COX-2 in the subject.

4. The whole coffee fruit extract for use of any one of claims 2 or 3, wherein the Aβ deposition in Alzheimer's disease is reduced.

5. The whole coffee fruit extract for use of any one of claims 2 to 4, wherein the method is for the treatment of a disease selected from Alzheimer's disease, Parkinson's disease, ischemic dementia, and Huntington's disease.

6. The whole coffee fruit extract for use of any one of claims 2 to 5, wherein the therapeutically effective amount of the whole coffee fruit extract is 50 to 400 mg/kg.

7. The whole coffee fruit extract for use of claim 6, wherein the therapeutically effective amount of the whole coffee fruit extract is 200 mg/kg.

## Patentansprüche

1. Verfahren zur Produktion von Extrakt der ganzen Kaffeefrucht, umfassend die Schritte:
(a) Bereitstellen von frisch gesammelter ganzer Kaffeefrucht, Trocknen der ganzen Kaffeefrucht, so dass der Wassergehalt 20 % oder weniger beträgt;
(b) Extrahieren der getrockneten ganzen Kaffeefrucht aus Schritt (a) mit 25 bis 30 % Ethanol in einem Verhältnis von Feststoff zu Flüssigkeit von 1:10 bis 1:5 bei 70 bis 80 °C für 3,5 bis 4 Stunden; und
(c) Gewinnen des Extrakts von Schritt (b),
wobei die ganze Kaffeefrucht Samen der Frucht einschließt.

2. Extrakt der ganzen Kaffeefrucht, der erhältlich ist nach dem Verfahren, welches die Schritte umfasst:
(a) Bereitstellen von frisch gesammelter ganzer Kaffeefrucht, Trocknen der ganzen Kaffeefrucht, so dass der Wassergehalt 20 % oder weniger beträgt;
(b) Extrahieren der getrockneten ganzen Kaffeefrucht aus Schritt (a) mit 25 bis 30 % Ethanol in einem Verhältnis von Feststoff zu Flüssigkeit von 1:10 bis 1:5 bei 70 bis 80 °C für 3,5 bis 4 Stunden; und
(c) Gewinnen des Extrakts von Schritt (b),
wobei die ganze Kaffeefrucht Samen der Frucht einschließt, zur Verwendung in einem Verfahren zum Reduzieren der Ablagerung von Aβ im Hippocampus eines Subjekts, das dessen bedarf, umfassend in dem Subjekt Verabreichen einer therapeutisch wirksamen Menge des ganzen Kaffeefruchtextrakts.

3. Extrakt der ganzen Kaffeefrucht zur Verwendung nach Anspruch 2, wobei das Verfahren die Expressionsniveaus von *p*-CREB und BDNF in dem Hippocampus und Cortex des Subjekts erhöht, und das Verfahren die Expressionsniveaus von *p*-eIF2α, BACE-1, Aβ, NLRP3, Caspase-1, IL-1β und COX-2 in dem Subjekt absenkt.

4. Extrakt der ganzen Kaffeefrucht zur Verwendung in einem der Ansprüche 2 oder 3, wobei die Aß-Ablagerung bei Morbus Alzheimer reduziert wird.

5. Extrakt der ganzen Kaffeefrucht zur Verwendung nach einem der Ansprüche 2 bis 4, wobei das Verfahren zur Behandlung einer Erkrankung ausgewählt aus Morbus Alzheimer, Morbus Parkinson, ischämischer Demenz und Morbus Huntington dient.

6. Extrakt der ganzen Kaffeefrucht zur Verwendung nach einem der Ansprüche 2 bis 5, wobei die therapeutisch effektive Menge des ganzen Kaffeefruchtextrakts 50 bis 400 mg/kg beträgt.

7. Extrakt der ganzen Kaffeefrucht zur Verwendung nach Anspruch 6, wobei die therapeutisch effektive Menge des ganzen Kaffeefruchtextrakts 200 mg/kg beträgt.

## Revendications

1. Méthode de production d'un extrait de fruits de café entiers, comprenant les étapes :
(a) de mise à disposition de fruits de café entiers fraîchement récoltés, de séchage des fruits de café entiers de manière à ce que la teneur en eau soit de 20% ou moins ;
(b) d'extraction des fruits de café entiers séchés de l'étape (a) avec de l'éthanol à 25 à 30% selon un rapport solide/liquide allant de 1:10 à 1:5 à de 70 à 80°C pendant de 3,5 à 4 heures ; et
(c) de récupération de l'extrait de l'étape (b) ;
dans laquelle les fruits de café entiers comportent les graines des fruits.

2. Extrait de fruits de café entiers pouvant être obtenu selon la méthode comprenant les étapes :
(a) de mise à disposition de fruits de café entiers fraîchement récoltés, de séchage des fruits de café entiers de manière à ce que la teneur en eau soit de 20% ou moins ;
(b) d'extraction des fruits de café entiers séchés de l'étape (a) avec de l'éthanol à 25 à 30% selon un rapport solide/liquide allant de 1:10 à 1:5 à de 70 à 80°C pendant de 3,5 à 4 heures ; et
(c) de récupération de l'extrait de l'étape (b) ;
dans lequel les fruits de café entiers comportent les graines des fruits ;
pour une utilisation dans une méthode destinée à la réduction du dépôt d'Aβ dans l'hippocampe d'un sujet en ayant besoin, comprenant l'administration au sujet d'une quantité thérapeutiquement efficace de l'extrait de fruits de café entiers.

3. Extrait de fruits de café entiers pour une utilisation selon la revendication 2, la méthode augmentant les taux d'expression de *p*-CREB et BDNF dans l'hippocampe et le cortex du sujet, et la méthode réduisant les taux d'expression de *p*-eIF2α, BACE-1, Aβ, NLRP3, caspase-1, IL-1β et COX-2 chez le sujet.

4. Extrait de fruits de café entiers pour une utilisation selon l'une quelconque des revendications 2 ou 3, le dépôt d'Aβ dans la maladie d'Alzheimer étant réduit.

5. Extrait de fruits de café entiers pour une utilisation selon l'une quelconque des revendications 2 à 4, la méthode étant destinée au traitement d'une maladie choisie parmi la maladie d'Alzheimer, la maladie de Parkinson, la démence ischémique, et la maladie de Huntington.

6. Extrait de fruits de café entiers pour une utilisation selon l'une quelconque des revendications 2 à 5, la quantité thérapeutiquement efficace de l'extrait de fruits de café entiers allant de 50 à 400 mg/kg.

7. Extrait de fruits de café entiers pour une utilisation selon la revendication 6, la quantité thérapeutiquement efficace de l'extrait de fruits de café entiers étant de 200 mg/kg.
